# EUROPÄISCHE PATENTSCHRIFT

(11) **EP 0 538 681 B1**
(45) Veröffentlichungstag und Bekanntmachung des Hinweises auf die Patenterteilung: **07.01.1998**
(21) Anmeldenummer: 92117174.0
(22) Anmeldetag: 08.10.1992
(51) Int. Cl.: C07C 43/178, C07C 41/14, C07C 41/26

(54) **Verfahren zur Herstellung von Monovinylethern**
Process for the preparation of monovinyl ethers
Procédé pour la préparation d'éthers monovinyliques

(30) Priorität: 22.10.1991 DE 4134807
(43) Veröffentlichungstag der Anmeldung: 28.04.1993
(73) Patentinhaber: BASF Aktiengesellschaft, 67063 Ludwigshafen (DE)
(72) Erfinder: Karcher, Michael, Dr., W-6830 Schwetzingen (DE); Henkelmann, Jochem, Dr., W-6700 Ludwigshafen (DE)

(56) Entgegenhaltungen:
- WO-A-90/03989
- CHEMICAL ABSTRACTS, vol. 99, no. 17, 24. Oktober 1983, Columbus, Ohio, US; abstract no. 139321t, 'HYDROXYALKYL VINYL ETHERS' Seite 547 ;Spalte 1 ; & PATENT ABSTRACTS OF JAPAN vol. 7, no. 114 (C-166)(1259) 18. Mai 1983 & JP-A-58 035 137 (ASAHI GLASS)
- TETRAHEDRON, (INCL. TETRAHEDRON REPORTS) Bd. 28, Nr. 2, Januar 1972, OXFORD GB Seiten 233 - 238 J. E. MCKEON & P. FITTON 'THE PALLADIUM(II) CATALYZED VINYL INTERCHANGE REACTION-II'

## Beschreibung

Die vorliegende Erfindung betrifft ein Verfahren zur Herstellung von Monovinylethern der allgemeinen Formel I 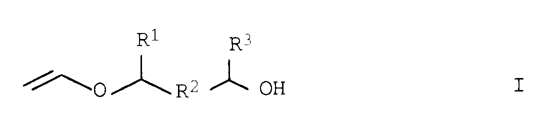 in der die Reste R¹ und R³ gleich oder verschieden sind und für Wasserstoff, C₁-C₂₀-Alkyl- und/oder C₃- bis C₁₀-Cycloalkylgruppen stehen und in der
R² eine C₁- bis C₂₀-Alkylen-, eine C₃- bis C₁₀-Cycloalkylen-, ein C₇- bis C₁₂-Aralkylen-, eine Phenylen- oder Naphthylengruppe ist.

Monovinylether von Diolen dienen zur Herstellung hochwertiger, strahlungshärtbarer Polyurethanlacke, wie sie in US-A 4 751 273 und US-A 4 775 732 beschrieben sind.

Bei der Herstellung dieser Monovinylether bedient man sich in der Regel der Reppe-Vinylierung (Liebigs Ann. Chem. 601, 81 (1956)), bei der die betreffenden Alkohole in Gegenwart starker Basen an Acetylen addiert werden. Als Nebenprodukt entstehen bei dieser Umsetzung, insbesondere bei deren Durchführung im industriellen Maßstab, Divinylether, für die es bislang nur eine beschränkte Verwendung gab. Da diese Divinylether auch nicht wirtschaftlich in die entsprechenden Monovinylether umgewandelt werden konnten, mußten die nicht absetzbaren Mengen dieser Nebenprodukte verbrannt oder anderweitig entsorgt werden.

Die Übertragung von Vinylgruppen aus Vinylgruppen-enthaltenden Verbindungen, insbesondere aus Vinylethern, auf Alkohole wird durch Quecksilber-, Cadmium- und Zinnsalze katalysiert (J. Org. Chem. 14, 1057 (1949)).

McKeon et al (Tetrahedron 28, 233 (1972)) beschreiben die Vinylgruppenübertragung von Vinylethern auf Alkohole, wobei sie Diacetato-(1,10-phenanthrolin)- sowie Diacetato-(2,2'-bipyridyl)-palladium(II)-Komplexe als Katalysatoren verwenden.

Gemäß EP-A 351 603 werden Ruthenium-Verbindungen als Katalysatoren zur Übertragung der Vinylgruppe von Vinylestern auf Alkohole und Hydroxycarbonsäureester benutzt.

JP-A 35137/1983 betrifft ein Umvinylierungsverfahren, bei dem ein Monovinylether eines Diols durch die Palladium-katalysierte Transvinylierung des Diols mit einem Vinylether eines einwertigen Alkohols erzeugt wird. Bei dieser Umsetzung entsteht als Nebenprodukt der entsprechende Divinylether.

WO 90/03989 betrifft ein Verfahren zur Vinylierung von Polyolen, die vorzugsweise mehr als 2 Hydroxylgruppen haben, durch deren Palladium-katalysierte Transvinylierung mit Vinylethern, deren Diol-Stammkörper von einem anderen Diol als dem zu vinylierenden Diol stammt. Als Folge dieser Verfahrensweise entstehen Gemische aus nicht-vinyliertem, einfach, zweifach und mehrfach vinyliertem Polyol, die als solche nicht trennbar sind und in Form des Gemisches zur Herstellung von Polyurethanen eingesetzt werden.

Der vorliegenden Erfindung lag daher die Aufgabe zugrunde, ein Verfahren zu entwickeln, das die wirtschaftliche Umwandlung der Divinylether in die betreffenden Monovinylether ermöglicht.

Dementsprechend wurde ein Verfahren zur Herstellung von Monovinylethern der allgemeinen Formel I 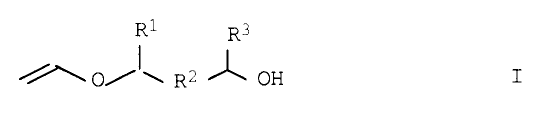 in der die Reste R¹ und R³ gleich oder verschieden sind und für Wasserstoff, C₁- bis C₁₀-Alkyl- und/oder C₃- bis C₁₀-Cycloalkylgruppen stehen und in der
R² eine C₁- bis C₂₀-Alkylen-, eine C₃- bis C₁₀-Cycloalkylen-, eine C₇- bis C₁₂-Aralkylen-, eine Phenylen- oder Naphthylengruppe ist, gefunden, das dadurch gekennzeichnet ist, daß man ein Diol der allgemeinen Formel II 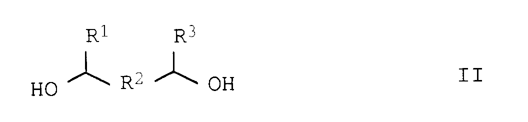 mit einem Divinylether der allgemeinen Formel III 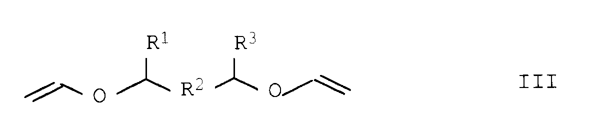 dessen Reste R¹, R² und R³ identisch mit denen des eingesetzten Diols II sind, in Gegenwart von Verbindungen der Platinmetalle umsetzt.

Bei der erfindungsgemäßen Umsetzung wird somit eine Vinylgruppe vom Divinylether auf das eingesetzte Diol übertragen. Da es sich hierbei um eine Gleichgewichtsreaktion handelt, wird zur Erzielung eines möglichst vollständigen Umsatzes eines der beiden Reaktanten, der andere Reaktant zweckmäßigerweise im Überschuß eingesetzt, im allgemeinen in einem 1,1- bis 5-fachen molaren Überschuß. Die Wahl, welcher der beiden Reaktanten im Überschuß eingesetzt wird, ist für den Erfolg des erfindungsgemäßen Verfahrens nicht kritisch und hängt allein von wirtschaftlichen Erwägungen ab, in der Regel wird jedoch das Diol im Überschuß bezüglich des Divinylethers eingesetzt.

Die Umsetzung wird im allgemeinen bei Temperaturen von -40 bis 150°C, vorzugsweise bei 0 bis 100°C und besonders bevorzugt bei 20 bis 80°C, bei Atmosphärendruck oder erhöhtem Druck, insbesondere unter dem Eigendruck des Reaktionssystems vorgenommen. Das Arbeiten unter vermindertem Druck ist ebenfalls möglich.

Die Umsetzung kann in Gegenwart eines unter den Reaktionsbedingungen inerten Lösungsmittels, beispielsweise eines Ethers, wie Diethylether, Diisopropylether, Methyl-tert.-Butylether, Tetrahydrofuran, Dioxan, eines Ketons, wie Acetophenon, eines Kohlenwasserstoffs, wie Benzol, Toluol oder Cyclohexan, eines Formamids, wie Dimethylformamid, eines sterisch gehinderten Alkohols, wie tert.-Butanol oder Neopentanol, eines Sulfoxids, wie Dimethylsulfoxid oder eines Sulfons, wie Dimethylsulfon oder Sulfolan, erfolgen. Ist mindestens einer der Reaktionspartner unter den Reaktionsbedingungen flüssig, so kann die Umsetzung vorteilhaft auch in Abwesenheit eines Lösungsmittels durchgeführt werden. Es kann dabei zweckmäßig sein, den flüssigen Reaktanten im Überschuß einzusetzen, so daß er quasi als Lösungsmittel für den festen Reaktionspartner dient.

Als Katalysatoren dienen im erfindungsgemäßen Verfahren Verbindungen der Platinmetalle, insbesondere Verbindungen des Platins, Palladiums, Rhodiums, Rutheniums und Iridiums. Besonders bevorzugt werden Palladium-Katalysatoren verwendet. Die Wertigkeitsstufe der Platinmetalle in den zur Umsetzung verwendeten Platinmetallverbindungen beträgt in der Regel + II für Platin und Palladium und 0 oder + III für Rhodium und Ruthenium.

Als Platinmetall-Verbindungen können die Salze oder Komplexe, insbesondere Chelatkomplexe der genannten Platinmetalle verwendet werden. Als solche Salze sind z.B. die Halogenide, insbesondere die Chloride, die Sulfate, Nitrate oder Gemische dieser Platinmetalle zu nennen. Vorzugsweise werden die Carboxylate , insbesondere C₁- bis C₁₀-Carboxylate, wie die Formiate, Acetate, Propionate, Butyrate, Valerianate, Decanoate, Succinate, Adipate, Acetoacetate und Benzoate der genannten Platinmetalle eingesetzt. Besonders bevorzugt werden die Acetate und Acetoacetate der genannten Platinmetalle benutzt. Beispielhaft für solche Platinmetallverbindungen seien Palladium(II)acetat, Rhodium(II)acetat oder Ruthenium(III)acetoacetat genannt.

Vorzugsweise werden die in erfindungsgemäßen Verfahren eingesetzten Platinmetallverbindungen, insbesondere die Platinmetallcarboxylate, noch zusätzlich mit Chelatliganden stabilisiert, wobei stickstoffhaltige Chelatliganden, wie 2,2-Bipyridin, 2,2-Naphthyridin oder Phenanthrolin, die mit unter den Reaktionsbedingungen inerten Substituenten substituiert sein können, bevorzugt sind. Vorteilhaft können aber auch Chelatkomplexe der Platinmetalle eingesetzt werden, deren Chelatliganden keinen Stickstoff enthalten, insbesondere Chelatliganden des Pentandiontyps, wie Acetylaceton. Als Beispiele für solche Chelat-stabilisierten Platinmetallkomplex-Katalysatoren seien Phenanthrolinpalladiumdichlorid, Phenanthrolinrhodiumdichlorid, 2,2-Bipyridinpalladiumdibromid, 2,2-Bipyridinpalladiumdichlorid, Phenanthrolinrutheniumtrichlorid, 2,2-Bipyridinrutheniumdichlorid, Phenanthrolinpalladiumdiacetat, Phenanthrolinrhodiumtriacetat, 2,2-Bipyridinpalladiumdiacetat, 2,2-Bipyridinrhodiumtriacetat, Phenanthrolinrutheniumtriacetat, Palladiumacetylacetonatodichlorid, Rutheniumacetylacetonatotrichlorid, Rhodiumacetylacetonatotrinitrat, Palladiumacetylacetonatosulfat sowie Platinacetylacetonatdichlorid genannt.

Die genannten Platinmetallkomplex-Katalysatoren lösen sich homogen im Reaktionsmedium, so daß die Umsetzung homogenkatalytisch durchgeführt werden kann. Es ist aber auch möglich im erfindungsgemäßen Verfahren heterogenisierte Platinmetallkomplex-Katalysatoren zu verwenden, insbesondere solche, in denen die Platinmetalle an polymeren Matrizes fixiert sind. Solche polymeren Matrizes können z. B. Harze, wie Styrol-Divinylbenzol-Harze oder Phenol-Formaldehyd-Harze sein, an die die betreffenden Chelatliganden gebunden sind, welche wiederum mit den Platinmetall-Verbindungen Komplexe bilden und diese derart quasi immobilisieren. Solche heterogenisierten, polymergebundenen Platinmetallkomplex-Katalysatoren sind beispielsweise nach dem Verfahren von Zhuangxu et al. (Reactive Polymers 9, 249 (1988)) erhältlich. Der Vorteil der Anwendung solcher heterogenisierter Katalysatoren liegt insbesondere in der leichteren und schonenderen Abtrennbarkeit des Katalysators vom Reaktionsgut. Dieser kann in einem Festbett angeordnet und von der Reaktionsmischung durchströmt werden oder aber auch im Reaktionsgut suspendiert und nach beendeter Umsetzung mechanisch, z.B. durch Filtration, abgetrennt werden.

Werden hingegen homogen im Reaktionsmedium gelöste Platinmetallverbindungen verwendet, so können diese nach beendeter Umsetzung vom Reaktionsprodukt z. B. mittels physikalischer Trennverfahren, beispielsweise durch Adsorption an geeignete Adsorbentien oder Kationenaustauscher, abgetrennt werden. Als anorganische Adsorbentien seien beispielhaft Aktivkohle, Calciumcarbonat, Bariumsulfat, Siliciumdioxid, Aluminiumoxide, Titandioxide und Zirkoniumdioxid genannt. Als Kationenaustauscher können handelsübliche Kationenaustauscherharze verwendet werden.

Eine weitere Möglichkeit der Trennung von Produkt und Katalysator besteht in der destillativen Abtrennung des Reaktionsproduktes, die gewünschtenfalls unter vermindertem Druck durchgeführt und in Kolonnen oder vorzugsweise in Verdampfern, insbesondere Dünnfilmverdampfern, vorgenommen werden kann, wobei gewünschtenfalls zur zusätzlichen Verminderung der thermischen Belastung von Produkt und Katalysator zusätzlich noch mit einem Inertgas, wie Stickstoff oder Argon, gestrippt werden kann. Bei einer derartigen destillativen Abtrennung des Reaktionsproduktes kann die Anwesenheit eines hochsiedenden Lösungsmittels im Reaktionsprodukt von Vorteil sein, insbesondere kann der im hochsiedenden Lösungsmittel zurückbleibende Katalysator wieder in die Umsetzung zurückgeführt und wiederverwendet werden. Als hochsiedende Lösungsmittel eignen sich zu diesem Zweck insbesondere Sulfoxide und Sulfone, wie Dimethylsulfoxid, Dimethylsulfon und Sulfolan (Tetrahydrothiophen-1,1-dioxid) sowie hochsiedende, oligomere Ethylenglykol-, Propylenglykolether und Butylenglykolether.

Die obengenannten Platinmetall-Katalysatoren können vorteilhaft in Mengen von 1 x 10⁻³ bis 1 x 10⁻⁵, vorzugsweise in Mengen von 5 x 10⁻³ bis 8 x 10⁻⁴ und besonders bevorzugt in Mengen von 1 x 10⁻⁴ bis 5 x 10⁻⁴ mol/Mol Einsatzstoff II oder III eingesetzt werden. Die Anwendung höherer Katalysatormengen ist möglich, allerdings aus Kostengründen in der Regel nicht bevorzugt.

Als Ausgangsmaterialien zur Herstellung der Monovinylether I dienen die Diole der Formel II 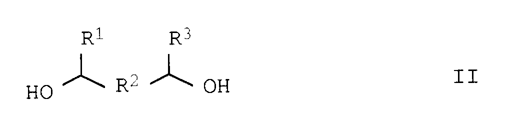 und die von Ihnen abgeleiteten Divinylether der Formel III 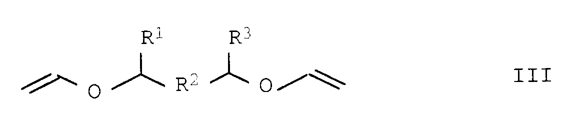 in denen die Reste R¹ und R³ gleich oder verschieden sind und für Wasserstoff, geradkettige oder verzweigte C₁- bis C₂₀-Alkyl- und/oder Cycloalkylgruppen stehen. Bevorzugte Reste R¹ und R³ sind Wasserstoff und/oder C₁- bis C₄-Alkylgruppen.

Der Rest R² kann eine C₁- bis C₂₀-, vorzugsweise eine C₁- bis C₄-Alkylengruppe, eine C₃- bis C₁₀-, vorzugsweise eine C₅- oder C₆-Cycloalkylengruppe, eine C₇- bis C₁₂-Alkylengruppe, eine Phenylen- oder Naphthylengruppe sein. Bevorzugte Reste R² sind die C₁- bis C₄-Alkylen- und C₅- oder C₆-Cycloalkylengruppe. Besonders bevorzugte Ausgangsmaterialien zur Herstellung der Monovinylether sind 1,4-Butandiole und 1,4-Bis(hydroxymethyl)cyclohexan der Formel IV 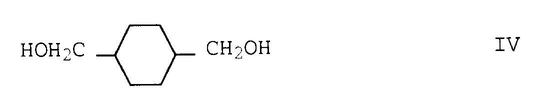 sowie die entsprechenden Divinylether dieser beiden Diole.

Zweckmäßigerweise setzt man jedoch das Diol II mit einem Divinylether III um, dessen Reste R¹, R² und R³ identisch mit denen des verwendeten Diols II sind.

1,4-Butandiol ist eine Grundchemikalie und wird industriell durch die Anlagerung von 2 Molekülen Formaldehyd an Acetylen und die anschließende Hydrierung des dabei entstandenen 2-Butin-1,4-diols hergestellt (vgl. K. Weissermel, H.-J. Arpe: Industrielle Organische Chemie, 2. Auflage, Seite 94 bis 96, Verlag Chemie, Weinheim 1978). 1,4-Bis-(hydroxymethyl)cyclohexan ist durch die Hydrierung von Terephthalsäuredialkylestern gemäß DE-AS 1 202 269 erhältlich. Auf analoge Weise können die übrigen Diole durch die katalytische Hydrierung der entsprechenden Dicarbonsäureester hergestellt werden. Die Divinylether sind wie eingangs erwähnt ein Nebenprodukt der Reppe-Vinylierung.

Nach dem erfindungsgemäßen Verfahren lassen sich Divinylether auf einfache und wirtschaftliche Weise und mit einer hohen Selektivität in die betreffenden Monovinylether umwandeln. Im erfindungsgemäßen Verfahren spielen die befürchteten Abscheidungen der Platinmetalle, infolge deren Reduktion zum Metall, praktisch keine Rolle.

### Beispiele

### Beispiel 1

142 g (1 Mol) Butandioldivinylether und 270 g (3 Mol) 1,4-Butandiol wurden mit 0,3 g (0,74 mMol) Phenanthrolinpalladiumdiacetat versetzt und anschließend 1 Stunde bei 60°C gerührt. Mit fortschreitender Reaktionsdauer wurde die anfangs 2-phasige Reaktionsmischung homogen. Nachdem der Divinylether zu 72 % umgesetzt war, wurde die Reaktion abgebrochen. In der rohen Reaktionsmischung konnten gaschromatographisch keine Nebenprodukte festgestellt werden. Die Selektivität der Umsetzung betrug 100 %. Das Rohprodukt wurde destillativ aufgearbeitet.

### Beispiel 2

196 g (1 Mol) Cyclohexandimethanoldivinylether und 432 g (Cyclohexandimethanol) (3 Mol) wurden mit 0,2 g (0,49 mMol) Phenanthrolinpalladiumdiacetat versetzt und 8 Stunden bei einer Temperatur von 60°C gerührt. Nach beendeter Umsetzung betrug der Umsatz bezüglich des Divinylethers 71 %. In der Reaktionsmischung konnten keine Nebenprodukte nachgewiesen werden. Die Selektivität betrug 100 %. Das Rohprodukt wurde destillativ aufgearbeitet.

## Patentansprüche

1. Verfahren zur Herstellung von Monovinylethern der allgemeinen Formel I 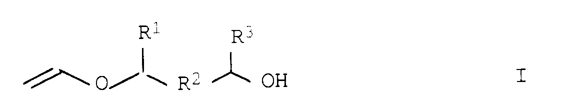 in der die Reste R¹ und R³ gleich oder verschieden sind und für Wasserstoff, C₁- bis C₂₀-Alkyl- und/oder C₃- bis C₁₀-Cyloalkylgruppen stehen und in der
R² eine C₁- bis C₂₀-Alkylen, eine C₃- bis C₁₀-Cycloalkylen-, eine C₇- bis C₁₂-Aralkylen-, eine Phenylen- oder Naphthylengruppe ist, dadurch gekennzeichnet, daß man ein Diol der allgemeinen Formel II 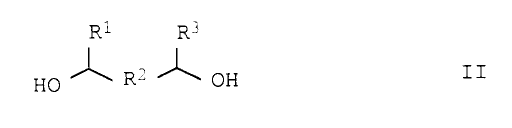 mit einem Divinylether der allgemeinen Formel III 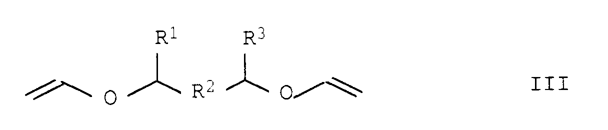 dessen Reste R¹, R² und R³ identisch mit denen des eingesetzten Diols II sind, in Gegenwart von Verbindungen der Platinmetalle umsetzt.

2. Verfahren nach Anspruch 1, dadurch gekennzeichnet, daß man die Umsetzung bei Temperaturen von -40 bis 150°C durchführt.

3. Verfahren nach Anspruch 1, dadurch gekennzeichnet, daß man als Katalysatoren Carboxylate der Platinmetalle einsetzt.

4. Verfahren nach Anspruch 1, dadurch gekennzeichnet, daß man als Katalysatoren mit Chelatliganden stabilisierte Carboxylate der Platinmetalle verwendet.

5. Verfahren nach Anspruch 1, dadurch gekennzeichnet, daß man als Katalysatoren Chelatkomplexe der Platinmetalle verwendet.

6. Verfahren nach Anspruch 1, dadurch gekennzeichnet, daß man polymere Katalysatoren einsetzt, in denen die Platinmetalle durch an Polymermatrizes fixierte Chelatliganden komplexiert sind.

7. Verfahren nach Anspruch 6, dadurch gekennzeichnet, daß man polymere Katalysatoren einsetzt, in denen Palladium der Oxidationsstufe +II durch an Polymermatrizes fixierte Chelatliganden komplexiert ist.

8. Verfahren nach Anspruch 1, dadurch gekennzeichnet, daß man als Platinmetalle Platin, Palladium, Rhodium, Iridium und/oder Ruthenium verwendet.

9. Verfahren nach Anspruch 1, dadurch gekennzeichnet, daß man 1,4-Bis-(hydroxymethyl) cyclohexan der Formel IV 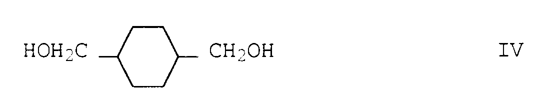 mit 1,4-Bis-(vinyloxymethylcyclohexan) der Formel V 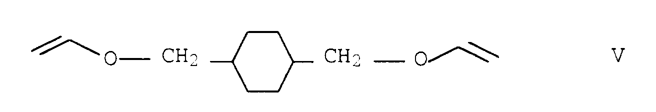 umsetzt.

10. Verfahren nach Anspruch 1, dadurch gekennzeichnet, daß man 1,4-Butandiol mit 1,4-Bis-(vinyloxy)butan der Formel VI 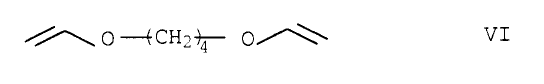 umsetzt.

## Claims

1. A process for the preparation of monovinyl ethers of the formula I 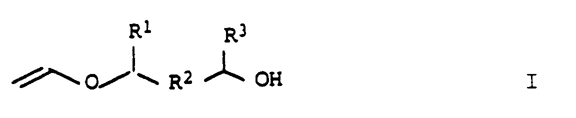 where R¹ and R³ are identical or different and are hydrogen, C₁-C₂₀-alkyl and/or C₃-C₁₀-cycloalkyl, and
R² is C₁-C₂₀-alkylene, C₃-C₁₀-cycloalkylene, C₇-C₁₂-aralkylene, phenylene or naphthylene,
which comprises reacting a diol of the formula II 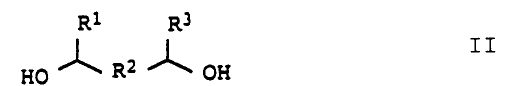 with a divinyl ether of the formula III 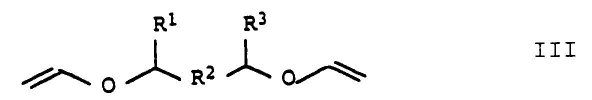 whose R¹, R² and R³ radicals are identical to those of the diol II employed,
in the presence of compounds of the platinum metal.

2. A process as claimed in claim 1, wherein the reaction is carried out at from -40 to 150°C.

3. A process as claimed in claim 1, wherein the catalyst employed is a platinum metal carboxylate.

4. A process as claimed in claim 1, wherein the catalyst used is a platinum metal carboxylate stabilized by chelate ligands.

5. A process as claimed in claim 1, wherein the catalyst used is a platinum metal chelate complex.

6. A process as claimed in claim 1, wherein a polymeric catalyst is employed in which the platinum metal is complexed by chelate ligands fixed to polymeric matrices.

7. A process as claimed in claim 6, wherein a polymeric catalyst is employed in which palladium of oxidation state +II is completed by chelate liquids fixed to polymeric matrices.

8. A process as claimed in claim 1, wherein the platinum metal used is platinum, palladium, rhodium, iridium and/or ruthenium.

9. A process as claimed in claim 1, wherein 1,4-bis-(hydroxymethyl)cyclohexane of the formula IV 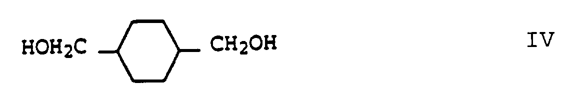 is reacted with 1,4-bis(vinyloxymethylcyclohexane) of the formula V

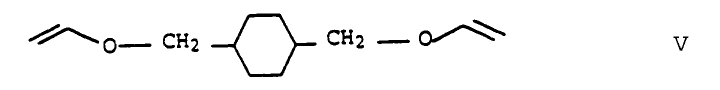

10. A process as claimed in claim 1, wherein 1,4-butanediol is reacted with 1,4-bis(vinyloxy)butane of the formula VI

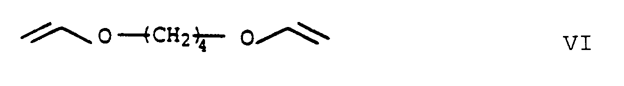

## Revendications

1. Procédé de préparation d'éthers monovinyliques de formule générale I 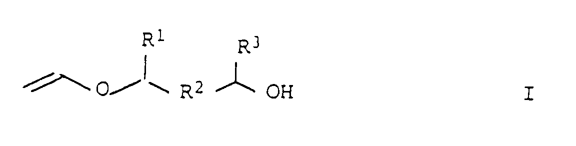 dans laquelle les restes R¹ et R³ sont identiques ou différents et sont mis pour des atomes d'hydrogène, des groupements alkyle en C₁-C₂₀ et/ou cycloalkyle en C₃-C₁₀ et dans laquelle
R² est un groupement alkylène en C₁-C₂₀, cycloalkylène en C₃-C₁₀, aralkylène en C₇-C₁₂ phénylène ou naphtylène, caractérisé en ce que l'on fait réagir un diol de formule générale II 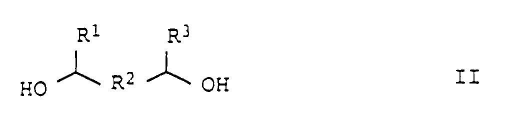
avec un éther divinylique de formule générale III 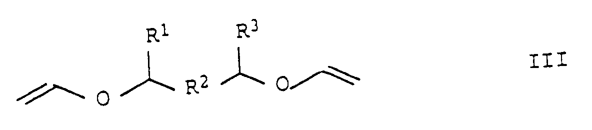 dont les restes R¹, R² et R³ sont identiques à ceux du diol II utilisé, en présence de composés de métaux du groupe du platine.

2. Procédé selon la revendication 1, caractérisé en ce que l'on effectue la réaction à des températures de -40 à 150°C.

3. Procédé selon la revendication 1, caractérisé en ce que l'on utilise des carboxylates de métaux du groupe du platine, en tant que catalyseurs.

4. Procédé selon la revendication 1, caractérisé en ce que l'on utilise des carboxylates de métaux du groupe du platine stabilisés par des ligands chélatants, en tant que catalyseurs.

5. Procédé selon la revendication 1, caractérisé en ce que l'on utilise des chélates de métaux du groupe du platine, en tant que catalyseurs.

6. Procédé selon la revendication 1, caractérisé en ce que l'on utilise des catalyseurs polymères, dans lesquels les métaux du groupe de platine sont complexés par des ligands chélatants fixés sur la matrice polymère.

7. Procédé selon la revendication 6, caractérisé en ce que l'on utilise des catalyseurs polymères, dans lesquels du palladium au degré d'oxydation + II est complexé par des ligands chélatants fixés sur la matrice polymère.

8. Procédé selon la revendication 1, caractérisé en ce que l'on utilise du platine, du palladium, du rhodium, de l'iridium et/ou du ruthénium, en tant que métaux du groupe du platine.

9. Procédé selon la revendication 1, caractérisé en ce que l'on fait réagir du 1,4-bis(hydroxyméthyl)cyclohexane de formule IV 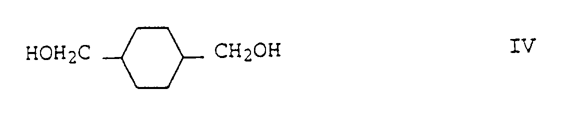 avec du 1,4-bis(vinyloxyméthyl)cyclohexane de formule V

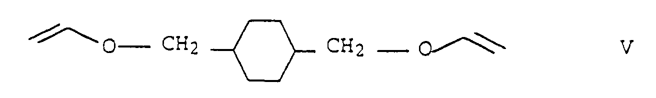

10. Procédé selon la revendication 1, caractérisé en ce que l'on fait réagir du 1,4-butanediol avec du 1,4-bis(vinyloxy)butane de formule VI
